(19) Europäisches Patentamt
European Patent Office
Office européen des brevets

(11) Veröffentlichungsnummer: **0 425 980 B1**

(12) **EUROPÄISCHE PATENTSCHRIFT**

(45) Veröffentlichungstag der Patentschrift: **27.07.94**

(51) Int. Cl.$^5$: **C07B 57/00**, C07D 493/04

(21) Anmeldenummer: **90120301.8**

(22) Anmeldetag: **23.10.90**

# (54) Verfahren zur Enantiomerentrennung von Benzopyranderivaten.

(30) Priorität: **03.11.89 DE 3936616**

(43) Veröffentlichungstag der Anmeldung:
**08.05.91 Patentblatt 91/19**

(45) Bekanntmachung des Hinweises auf die Patenterteilung:
**27.07.94 Patentblatt 94/30**

(84) Benannte Vertragsstaaten:
**AT BE CH DE DK ES FR GB GR IT LI LU NL SE**

(56) Entgegenhaltungen:
**EP-A- 0 383 316**
**DE-A- 1 948 368**
**GB-A- 2 204 868**

(73) Patentinhaber: **MERCK PATENT GmbH**
**Postfach,**
**Frankfurter Strasse 250**
**D-64271 Darmstadt(DE)**

(72) Erfinder: **Devant, Ralf, Dr.**
**Im Hilsbruch 87**
**W-6100 Darmstadt(DE)**
Erfinder: **Gericke, Rolf, Dr.**
**Mozartstrasse 19**
**W-6104 Seeheim(DE)**

EP 0 425 980 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Enantiomerentrennung von 3,4-Epoxy-3,4-dihydro-2,2-dimethyl-3-R-2H-1-benzopyran-6-carbonitrilen (2,2-Dimethyl-3-R-3,4-epoxy-6-cyan-chromanen; "I")

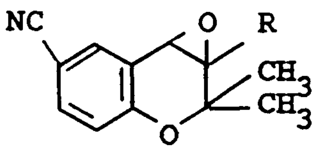

I,

worin R H oder $CH_3$ bedeutet.

Die Verbindungen I umschließen das bevorzugte 3,4-Epoxy-3,4-dihydro-2,2-dimethyl-2H-1-benzopyran-6-carbonitril (Ia) und das 3,4-Epoxy-3,4-dihydro-2,2,3-trimethyl-2H-1-benzopyran-6-carbonitril (Ib).

Racemisches Ia und seine beiden Enantiomeren, (+)-Ia und (-)-Ia sind aus der GB-A-2 204 868 bekannt. Nach den dort gemachten Angaben sind (+)-Ia und (-)-Ia erhältlich, indem man trans-3-Brom-4-hydroxy-2,2-dimethyl-2H-1-benzopyran-6-carbonitril mit (-)-Camphansäure verestert, das erhaltene Diastereomerengemisch chromatographisch trennt und die beiden Diastereomeren alkalisch verseift, wobei gleichzeitig unter HBr-Abspaltung der Epoxidring geschlossen wird.

Racemisches Ib (F. 118°) und sein beiden Enantiomeren (+)-Ib und (-)-Ib sind analog aus trans-3-Brom-4-hydroxy-2,2,3-trimethyl-2H-1-benzopyran-6-carbonitril erhältlich, das seinerseits in konventioneller Weise aus 3-Acetyl-4-hydroxy-benzonitril über 2,2-Dimethyl-6-cyan-4-chromanon (F. 119-120°), 2,2-Dimethyl-3-methylen-6-cyan-4-chromanon, 2,2,3-Trimethyl-6-cyan-4-chromanol und 2,2,3-Trimethyl-6-cyan-2H-chromen (F. 55°) herstellbar ist.

Die angegebenen Verfahren der chemischen Enantiomerentrennung haben in der Praxis große Nachteile, denn es sind teure Hilfsreagenzien erforderlich, und die Trennung erfordert zwei zusätzliche chemische Umsetzungen.

Der Erfindung lag die Aufgabe zugrunde, ein Verfahren zur Enantiomerentrennung von I zur Verfügung zu stellen, das die Nachteile dieser Verfahren nicht oder nur in geringerem Maße besitzt. Diese Aufgabe wurde durch die Auffindung des vorliegenden Verfahrens der "Cristallization by Entrainment" gelöst.

Gegenstand der Erfindung ist demnach ein Verfahren zur Enantiomerentrennung von I, dadurch gekennzeichnet, daß man racemisches I zusammen mit einer geringen Menge (-)-I [bzw. (+)-I] in einem inerten Lösungsmittel oder Lösungsmittelgemisch löst, die Lösung mit (-)-I [bzw. (+)-I] animpft, das ausgefallene (-)-I [ bzw.(+)-I] isoliert, in dem Filtrat weiteres racemisches I löst, mit (+)-I [bzw. (-)-I] animpft, das ausgefallene (+)-I [bzw. (-)-I] isoliert und gewünschtenfalls diesen Kristallisationszyklus ein oder mehrere Male wiederholt.

Dieses Entrainment-Verfahren kann in der Regel zur Spaltung racemischer Verbindungen nicht verwendet werden. Daher ist es überraschend, daß es im Falle von I mit Erfolg angewendet werden kann.

Als Lösungsmittel eignen sich Ether wie Tetrahydrofuran oder Methyl-tert.-butylether, niedere Alkohole, die 1-4 C-Atome enthalten, insbesondere Isopropanol, oder Gemische von niederen Alkoholen mit Kohlenwasserstoffen, z.B. Isopropanol/Hexan, oder von halogenierten Kohlenwasserstoffen mit Kohlenwasserstoffen, z.B. Dichlormethan/Hexan. Tetrahydrofuran ist besonders bevorzugt.

Im einzelnen löst man racemisches I zusammen mit etwa 1,5-2,5 Gew.% (-)- oder (+)-I zweckmäßig in der Hitze in etwa 1-2 Volumina (z.B. ml, bezogen auf 1 g I) Tetrahydrofuran, kühlt ab und impft mit einer Spur (z.B etwa 0,004-0,1 gew.%) reinem (-)-I [oder (+)-I]. Das auskristallisierte (-)-I [oder (+)-I] wird isoliert, zweckmäßig abfiltriert. Das Filtrat wird zweckmäßig mit einer weiteren Menge des Racemats versetzt, die der Menge des vorher abfiltrierten Enantiomeren entspricht, und das zugesetzte Material wird in der Wärme gelöst. Erneutes Abkühlen und Animpfen mit (+)-I [oder (-)-I], also dem anderen Enantiomeren, bewirkt eine Auskristallisation von (+)-I [oder (-)-I], welches man ebenfalls isoliert, zweckmäßig abfiltriert. Dieser Kristallisationszyklus kann ein oder mehrere Male wiederholt werden, indem man in dem zuletzt erhaltenen Filtrat weiteres racemisches I auflöst, durch Abkühlen und Animpfen weiteres (-)-I [oder (+)-I] zur Kristallisation bringt, im Filtrat davon erneut racemisches I auflöst, durch Abkühlen und Animpfen weiteres (+)-I [oder (-)-I] gewinnt usw.

Diese Ausführungsform zeichnet sich dadurch aus, daß im Gegensatz zu üblichen Kristallisationsverfahren aus sehr konzentrierter Lösung Kristallisiert wird.

Alle Temperaturen sind in °C angegeben.

Beispiel 1

In 700 ml Tetrahydrofuran werden bei 35° 475 g racemisches Ia und 5 g reines (-)-Ia gelöst. Unter Rühren läßt man auf 17° abkühlen und impft mit 20 mg reinem (-)-Ia. Nach 4 Std. werden 29 g (-)-Ia abfiltriert; optische Reinheit 92 % ee.

Zur Mutterlauge werden unter Erwärmen 29 g racemisches Ia zugegeben und durch Rühren gelöst. Nach erneutem Abkühlen auf 17° impft man mit 20 mg reinem (+)-Ia. Nach 4 Std. werden 31 g (+)-Ia abfiltriert; optische Reinheit 90 % ee.

Der Kristallisationszyklus läßt sich mehrere Male wiederholen.

Durch nochmaliges Umkristallisieren der so erhaltenen Enantiomere aus Isopropanol erhält man Produkte mit einer Enantiomerenreinheit von ≥ 99 % ee:
(-)-Enantiomer, F. 141-142°; $[\alpha]_D^{25}$ -92,5° (c = 1 in Methanol)
(+)-Enantiomer, F. 141-142°; $[\alpha]_D^{25}$ +91,7° (c = 1 in Methanol)
Die optische Reinheit kann mittels "Differential Scanning Calorimetry" ("DSC") bestimmt werden oder auch nach Umsetzung mit (S)-1-Phenylethylamin zu den diastereomeren 3,4-Dihydro-3-hydroxy-2,2-dimethyl-4-(1-phenylethylamino)-2H-benzopyran-6-carbonitrilen HPLC-chromatographisch.

Beispiel 2

Analog Beispiel 1 erhält man aus racemische Ib
(-)-Ib, F. 153-155°; $[\alpha]_D^{25}$ -135,2° (c = 1 in Methanol), sowie
(+)-Ib, F. 153-155°; $[\alpha]_D^{25}$ +135,2° (c = 1 in Methanol).

**Patentansprüche**

1. Verfahren zur Enantiomerentrennung von 3,4-Epoxy-3,4-dihydro-2,2-dimethyl-3-R-2H-1-benzopyran-6-carbonitrilen der Formel I

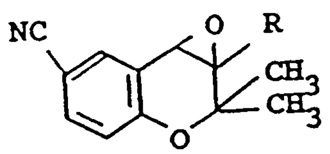

I

worin R H (Ia) oder $CH_3$ (Ib) bedeutet, dadurch gekennzeichnet, daß man racemisches I zusammen mit einer geringen Menge (-)-I [bzw. (+)-I] in einem inerten Lösungsmittel oder Lösungsmittelgemisch, bestehend aus Ethern, wie Tetrahydrofuran oder Methyl-tert.-butylether, niederen Alkoholen, die 1-4 C-Atome enthalten, wie Isopropanol, oder Gemischen von niederen Alkoholen mit Kohlenwasserstoffen, wie Isopropanol/Hexan, oder von halogenierten Kohlenwasserstoffen mit Kohlenwasserstoffen, wie Dichlormethan/ Hexan, löst, die Lösung mit (-)-I [bzw. (+)-I] animpft, das ausgefallene (-)-I [bzw. (+)-I] isoliert, in dem Filtrat weiteres racemisches I löst, mit (+)-I [bzw. (-)-I] animpft, das ausgefallene (+)-I [bzw. (-)-I] isoliert und gewünschtenfalls diesen Kristallisationszyklus ein oder mehrere Male wiederholt, wobei sämtliche Zyklen einer Trennung entweder mit Substanzen des Strukturtyps Ia oder Ib durchgeführt werden müssen.

2. Verfahren nach Anspruch 1, dadurch gekennzeichnet, daß man als Lösungsmittel Tetrahydrofuran verwendet.

**Claims**

1. Process for the resolution of enantiomers of 3,4-epoxy-3,4-dihydro-2,2-dimethyl-3-R-2H-1-benzopyran-6-carbonitriles

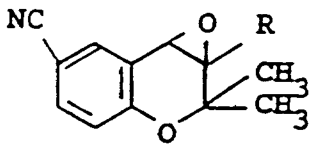

I,

of the formula I in which R is H (Ia) or $CH_3$ (Ib), characterized in that racemic I is dissolved in an inert solvent or solvent mixture, consisting of ethers, such as tetrahydrofuran or methyl tert.-butyl ether, lower alcohols which contain 1 - 4 C atoms such as isopropanol, or mixtures of lower alcohols with hydrocarbons such as isopropanol/hexane or of halogenated hydrocarbons such as dichloromethane/hexane, together with a small amount of (-)-I [or (+)-I], the solution is seeded with (-)-I [or (+)-I], the precipitated (-)-I [or (+)-I] is isolated, further racemic I is dissolved in the filtrate, seeded with (+)-I [or (-)-I], the precipitated (+)-I [or (-)-I] is isolated and, if desired, this crystallization cycle is repeated one or more times, all the cycles of one resolution having to be carried out with substances of either structural type Ia or Ib.

**2.** Process according to Claim 1, characterized in that tetrahydrofuran is used as solvent.

## Revendications

**1.** Procédé de séparation des énantiomères des 3,4-époxy-3,4-dihydro-2,2-diméthyl-3-R-2H-1-benzopyrane-6-carbonitriles de formule I

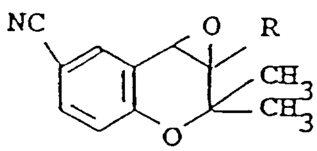

I

dans laquelle R représente un atome d'hydrogène (Ia) ou $CH_3$ (Ib), caractérisé en ce que l'on dissout le mélange racémique I avec une faible quantité de (-)- I [ou de (+) -I] dans un solvant inerte ou un mélange de solvants constitué d'éthers tel que le tétrahydrofuranne ou l'éther méthyl-tert.-butylique, des alcools inférieurs contenant de 1 à 4 atomes de carbone tel que l'isopropanol ou des mélanges d'alcools inférieurs avec des hydrocarbures tel que le mélange isopropanol/hexane ou des mélanges d'hydrocarbures halogénés avec des hydcocarbures tel que le mélange dichlorométhane/hexane, que l'on ensemence la solution par le composé (-)-I[ou (+)-I], que l'on sépare le composé (-)-I[ou (+)-I] précipité, que l'on dissout dans le filtrat une quantité supplémentaire de mélange racémique I, que l'on ensemence par (+)-I [ou (-)-I], que l'on sépare le composé (+)-I [ou (-)-I] précipité et que l'on répète éventuellement ce cycle de cristallisation une ou plusieurs fois, tous les cycles d'une séparation devant être effectués soit avec des substances de structure de type Ia, soit de structure de type Ib.

**2.** Procédé selon la revendication 1, caractérisé en ce que l'on utilise comme solvant du tétrahydrofuranne.